# EUROPÄISCHE PATENTSCHRIFT

(11) **EP 2 233 097 B1**
(45) Veröffentlichungstag und Bekanntmachung des Hinweises auf die Patenterteilung: **08.02.2012**
(21) Anmeldenummer: 09156346.0
(22) Anmeldetag: 26.03.2009
(51) Int. Cl.: A61B 17/88

(54) **Instrumentensatz zum Einsetzen eines Stabilisierungssystems in die Wirbelsäule eines Körpers**
Instrument set for inserting a stabilisation system into the spine of a body
Ensemble d'instruments destinés à installer un système de stabilisation dans la colonne vertébrale d'un corps

(43) Veröffentlichungstag der Anmeldung: 29.09.2010
(73) Patentinhaber: Spinelab AG, 8406 Winterthur (CH)
(72) Erfinder: Braunschweiler, Reto, 8413, Neftenbach (CH); Fischli, Simon, Ottawa Ontario K1S 2A1 (CA)
(74) Vertreter: BOVARD AG

(56) Entgegenhaltungen:
- WO-A-2006/130589
- US-A1- 2006 079 909
- US-A1- 2006 111 712
- US-A1- 2008 154 277

## Beschreibung

Die vorliegende Erfindung bezieht sich auf einen Instrumentensatz zum Einsetzen eines Stabilisierungssystem, bestehend aus Verankerungselementen, die jeweils eine Schraubenhalterung und einen U-förmigen Kopfteil aufweisen, in welchen Kopfteil ein Stab zur Stabilisierung einsetzbar ist und mit einer Verschlusseinrichtung verschliessbar und verriegelbar ist, in die Wirbelsäule eines Körpers, umfassend Mittel zum Einsetzen der Verankerungselemente in den jeweiligen Knochen- oder Wirbelkörper, Mittel einschliesslich einer Zange zum Einsetzen des Stabes zur Stabilisierung in die eingesetzten Verankerungselemente und Mittel zum Aufsetzen und Verspannen der Verschlusseinrichtungen auf die Verankerungselemente.

Verankerungselemente, in welche ein Stab eingesetzt werden kann, welcher Stab dann über eine auf das Verankerungselement aufgesetzte Verschlusseinrichtung im Verankerungselement gehalten wird, sind in vielfältiger Weise aus dem Stand der Technik bekannt. Aus Verankerungselementen, Verschlusseinrichtungen und Stab bestehende Stabilisierungssysteme dienen zur Stabilisierung der Wirbelsäulen von Patienten, welche Wirbelsäulen starke Schäden aufweisen. Hierzu wird in eine Anzahl von Wirbelkörpern jeweils ein Verankerungselement eingesetzt, beispielsweise durch Verschraubung, in die Kopfteile dieser Verankerungselemente wird ein Stab eingelegt, welcher dann jeweils mit dem entsprechenden Verankerungselement verbunden wird, wozu die Verschlusseinrichtungen verwendet werden. Hierbei können zwei unterschiedliche Stabilisierungsformen erreicht werden, beim Einsatz eines starren Stabes erreicht man eine Versteifung der betroffenen Wirbelkörper, beim Einsatz eines elastischen Stabes wird eine stützende Stabilisierung der Wirbelkörper erreicht, hierbei wird eine gewisse Beweglichkeit zwischen den einzelnen Wirbelkörpern zugelassen.

Eine bekannte Art von Verankerungselementen sind beispielsweise Pedikelschrauben. Eine Pedikelschraube für ein Stabilisierungssystem mit Verschlusseinrichtung und Stab ist beispielsweise der europäischen Patentanmeldung mit der Anmeldung Nr. 07150489.8 (Veröffentlichungs-Nr. EP 20 74 957 A1) entnehmbar. Die Pedikelschrauben werden über ein entsprechendes Werkzeug in den jeweils vorbereiteten Wirbelkörper eingeschraubt, der Stab wird in die U-förmigen Kopfteile dieser Schrauben eingesetzt, auf jede Schraube wird eine Verschlusseinrichtung aufgesetzt, der Verriegelungsteil wird in die verriegelte Position gebracht, die Spannschraube wird angezogen. Zum Einsetzen dieses Stabilisierungssystems in die Wirbelsäule eines Körpers werden Instrumente als Hilfsmittel verwendet. Hierbei ist es von ausschlaggebender Bedeutung, dass das in die Wirbelsäule eingesetzte Stabilisierungssystem absolut korrekt eingesetzt wird, um ausschliessen zu können, dass durch unkorrekte Handhabung sich das Stabilisierungssystem in einem späteren Zeitpunkt, beispielsweise durch Verschieben des Stabes in den Pedikelschrauben, verändern kann, was unabsehbare Folgen haben könnte.

Aus der Veröffentlichung US 2006/079909 A1 ist ein gattungsgemäßer Instrumentensatz bekannt. Er besteht aus Verankerungselementen mit einer Schraubenhalterung und einem U-förmigen Kopfteil sowie aus einem Stab, der in einen Kopfteil einsetzbar ist. Weiterhin besteht er aus einer Zange zum Greifen des U-förmigen Kopfteils sowie einem Werkzeug zum Einsetzen des Stabes. Die Zange und das Werkzeug weisen Führungsmittel auf, die miteinander zusammenwirken.

Die Aufgabe der vorliegenden Erfindung besteht somit darin, einen Instrumentensatz zum Einsetzen eines Stabilisierungssystems in die Wirbelsäule eines Körpers zu schaffen, welches ermöglicht, dass insbesondere der Einsatz des Stabes in die Verankerungselemente, das Aufsetzen der Verschlusseinrichtung auf die Verankerungselemente und das Verriegeln der Verschlusseinrichtung auf den Verankerungselementen und das Verspannen des Stabes in einfacher und sicherer Weise ausgeführt werden können.

Erfindungsgemäss erfolgt die Lösung dieser Aufgabe dadurch, dass die Mittel zum Einsetzen der Verankerungselemente einen Kopf und einen Schaft aufweisen, dass der Kopf derart ausgebildet ist, dass er in den U-förmigen Kopfteil des Verankerungselements einsteckbar ist, dass der Schaft mit ersten Führungsmitteln ausgestattet ist, entlang welchen die mit zweiten Führungsmitteln ausgerüstete Zange geführt auf den Kopfteil des Verankerungselements aufsetzbar und mit diesem koppelbar ist, und dass die Mittel zum Einsetzen des Stabes und die Mittel zum Aufsetzen und Verspannen der Verschlusseinrichtung mit weiteren Führungsmitteln ausgestattet sind, die mit den zweiten Führungsmitteln der Zange zusammenwirken.

Mit einem derartig ausgestalteten Instrumentensatz zum Einsetzen eines Stabilisierungssystems in eine Wirbelsäule eines Körpers ist jedes einzelne Instrument, das zum Einsetzen dieses Stabilisierungssystems eingesetzt wird, bezüglich des Kopfteils des Verankerungselements immer geführt, die Platzierung des Stabes und das Anbringen der Verschlusseinrichtung auf dem Verankerungselement sowie deren Verriegelung und Verspannen des Stabes erfolgt ausschliesslich über eindeutig geführte und positionierte Instrumente, wodurch Fehlmanipulationen mit den erwähnten folgenschweren Auswirkungen praktisch ausgeschlossen werden können.

In vorteilhafter Weise sind die ersten Führungsmittel an einer Führungshülse angebracht, welche Führungshülse auf den Schaft der Mittel zum Einschrauben aufschiebbar ist. Dadurch wird eine einfache Ausgestaltung der Mittel zum Einsetzen der Verankerungselemente erreicht.

In vorteilhafter Weise ist die Zange mit Backen versehen, welche die beiden Schenkel des U-förmigen Kopfteils aussenseitig greifen, und welche mit Nocken versehen sind, die in entsprechend an den beiden Schenkeln angebrachte Ausnehmungen eingreifen und dadurch die Zange an das Verankerungselement angekoppelt ist. Dadurch wird einerseits ein sicheres Festhalten der Zange am Verankerungselement erhalten, andererseits ist der Bereich des Verankerungselements, welcher den Stab und die Verschlusseinrichtung aufnehmen muss, frei zugänglich.

In vorteilhafter Weise sind die Backen der Zange bezüglich der Schwenkachse der Zange in Schwenkachsenrichtung versetzt angeordnet, wodurch der Zugang zum Verankerungselement in axialer Richtung freigehalten werden kann.

In vorteilhafter Weise ist die Zange in der mit dem Verankerungselement gekoppelten Position über Arretiermittel arretierbar, wodurch gewährleistet ist, dass die Verbindung zwischen Verankerungselement und darauf aufgesetzter Zange nicht unabsichtlich gelöst werden kann.

Eine weitere vorteilhafte Ausgestaltung der Erfindung besteht darin, dass die Mittel zum Einsetzen des Stabes in das Verankerungselement aus einer Stange gebildet sind, an deren vorderen Ende ein Einpressteil und deren hinteren Ende ein Handgriff angebracht ist, und dass die Stange mit dritten Führungsmitteln versehen ist, welche mit den zweiten Führungsmitteln der Zange zusammenwirken. Dadurch wird neben der einfachen Handhabung auch gewährleistet, dass sich die Mittel zum Einsetzen des Stabes bezüglich des Verankerungselements immer in einer exakt geführten Position befinden.

In vorteilhafter Weise sind die Mittel zum Aufsetzen und Verspannen der Verschlusseinrichtung aus einer Hülse gebildet, an deren vorderen Ende ein Aufnahmeteil für die Aufnahme der Verschlusseinrichtung angebracht ist, und welche Hülse mit vierten Führungsmitteln ausgestattet ist, die mit den zweiten Führungsmitteln der Zange zusammenwirken. Auch dadurch ist gewährleistet, dass die Verschlusseinrichtung mit Hilfe der Hülse genau auf dem Verankerungselement positioniert werden kann.

In vorteilhafter Weise besteht die Verschlusseinrichtung aus einem Einführteil, einem Verriegelungsteil und einer Spannschraube, welche Verschlusseinrichtung in optimaler Weise auf das Verankerungselement aufsetzbar, mit dieser verriegelbar und spannbar ist.

In vorteilhafter Weise ist der Aufnahmeteil mit schlitzförmigen Ausnehmungen versehen, in welche der Verriegelungsteil der Verschlusseinrichtung eindrehbar ist und dadurch die Verschlusseinrichtung im Aufnahmeteil gehalten ist, was ein unbeabsichtigtes Verlieren der Verschlusseinrichtung aus dem Aufnahmeteil unterbindet.

Eine weitere vorteilhafte Ausgestaltung der Erfindung besteht darin, dass in die Hülse ein Drehelement eingesetzt ist, dessen vorderes Ende mit Vorsprüngen ausgestattet ist, welche im eingesetzten Zustand der Verschlusseinrichtung in den Aufnahmeteil über den Verriegelungsteil vorstehend sind und dadurch der Verriegelungsteil bezüglich des Einführteils durch Verdrehen des Drehelementes verdrehbar ist, was eine positionsgenaue und einfache Verriegelung der Verschlusseinrichtung mit dem Verankerungselement ermöglicht.

Um eine einfache Handhabung der Hülse und des Drehelementes zu ermöglichen, ist die Hülse an deren hinteren Ende mit einem Handgriff versehen, während das Drehelement am über die Hülse vorstehenden hinteren Ende mit einem Drehknopf ausgestattet ist.

Eine weitere vorteilhafte Ausgestaltung der Erfindung besteht darin, dass das Drehelement eine durchgehende Längsbohrung aufweist, und dass in die durchgehende Längsbohrung ein Schraubenzieher einsteckbar ist, mit welchem die Spannschraube der Verschlusseinrichtung verdrehbar ist. Auch dadurch ist gewährleistet, dass das Einsetzen des Schraubenziehers und das Spannen der Spannschraube der Verschlusseinrichtung optimal geführt ausgeführt werden kann.

Eine Ausführungsform der Erfindung wird nachfolgend anhand der beiliegenden Zeichnung beispielhaft näher erläutert, wobei im dargestellten Ausführungsbeispiel das Verankerungselement als Pedikelschraube ausgebildet ist. Selbstverständlich wären auch anders ausgestaltete Verankerungselemente denkbar.

Es zeigt:
Fig. 1 in räumlicher Darstellung die Mittel zum Einschrauben der Pedikelschraube in einen Wirbelkörper;
Fig. 2 in räumlicher Darstellung die Mittel zum Einschrauben der Pedikelschraube während des Einschraubvorgangs;
Fig. 3 in räumlicher Darstellung den Schaft der Mittel zum Einschrauben der Pedikelschraube und die Führungshülse, die auf den Schaft aufgesetzt werden kann;
Fig. 4 in räumlicher Darstellung der Schaft mit aufgesetzter Führungshülse und die auf die Führungshülse aufschiebbare Zange;
Fig. 5 in räumlicher Darstellung die auf die Führungshülse aufgesetzte Zange im eingekoppelten Zustand mit der Pedikelschraube;
Fig. 6 eine räumliche vergrösserte Darstellung der Zangenbacken und der Pedikelschraube mit aufgesetztem Schaft;
Fig. 7 in räumlicher Darstellung die Mittel zum Einsetzen des Stabes in die Pedikelschraube im in die Zange eingesetzten Zustand;
Fig. 8 in räumlicher Darstellung zwei Wirbelkörper mit eingesetzter Pedikelschraube, jeweils aufgesetzter Zange und eingesetzten Mitteln zum Einsetzen des Stabes in die Pedikelschrauben;
Fig. 9 eine Ansicht auf ein Instrument zur Distrahierung zweier benachbarter Wirbelkörper;
Fig. 10 in räumlicher Darstellung die auf die Pedikelschraube aufgesetzte Zange, die Verschlusseinrichtung und die Mittel zum Aufsetzen und Verspannen der Verschlusseinrichtung;
Fig. 11 in räumlicher Darstellung die Verschlusseinrichtung;
Fig. 12 in räumlicher Darstellung der Aufnahmeteil der Mittel zum Aufsetzen und Verspannen der Verschlusseinrichtung und die darin einsetzbare Verschlusseinrichtung;
Fig. 13 in räumlicher Darstellung der Aufnahmeteil mit darin eingesetzter Verschlusseinrichtung;
Fig. 14 in räumlicher Darstellung die auf die Pedikelschraube aufgesetzte Zange, die Mittel zum Aufsetzen und Verspannen der Verschlusseinrichtung und den in die Mittel zum Aufsetzen und Verspannen der Verschlusseinrichtung einsteckbaren Schraubenzieher;
Fig. 15 in räumlicher Darstellung die in die Zange eingeführten Mittel zum Aufsetzen und Verspannen der Verschlusseinrichtung;
Fig. 16 in räumlicher Darstellung den in die Mittel zum Aufsetzen und Verspannen der Verschlusseinrichtung eingesetzten Schraubenzieher zum Anziehen der Spannschraube;
Fig. 17 eine Schnittdarstellung der Mittel zum Aufsetzen und Verspannen der Verschlusseinrichtung; und
Fig. 18 in räumlicher Darstellung das in die Wirbelkörper eingesetzte Stabilisierungssystem.

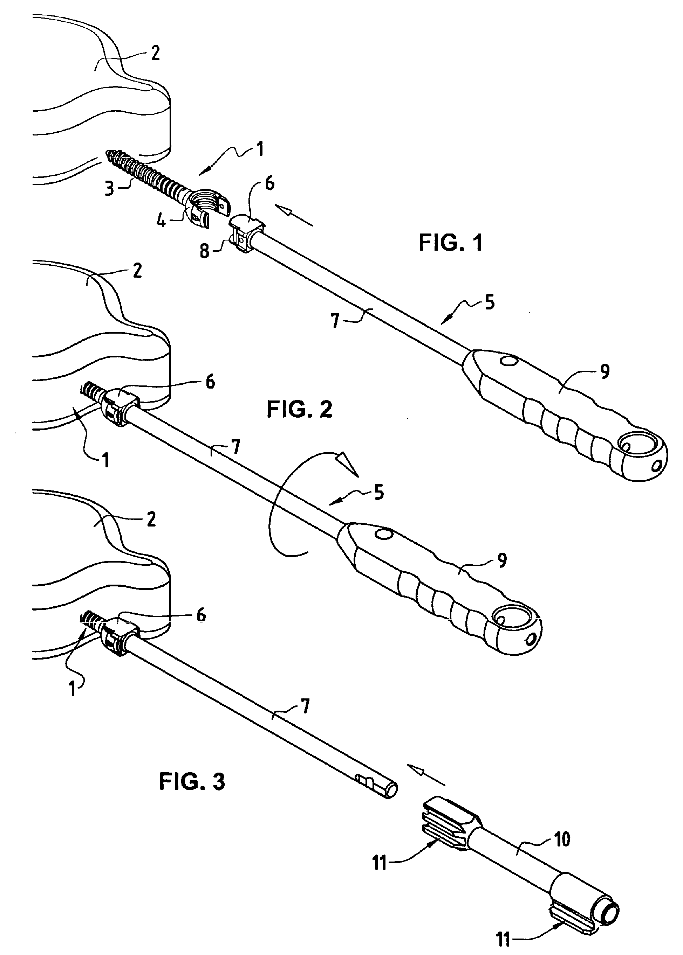

Aus Fig. 1 ist die Pedikelschraube 1 ersichtlich, welche in den Wirbelkörper 2 eingeschraubt werden soll. In bekannter Weise weist die Pedikelschraube 1 einen Einschraubteil 3 und einen U-förmigen Kopfteil 4 auf. Zum Einschrauben der Pedikelschraube 1 werden Mittel 5 verwendet, die aus einem Kopf 6 und einem Schaft 7 bestehen. Der Kopf 6 ist hierbei derart ausgestaltet, dass er in den U-förmigen Kopfteil 4 der Pedikelschraube 1 passend eingesteckt werden kann. Insbesondere ist der Kopf 6 mit Führungsrippen 8 ausgestattet, wodurch die auf die Mittel zum Einschrauben 5 aufgesetzte Pedikelschraube 1 koaxial zum Schaft 7 ausgerichtet ist und vom Kopf 6 festgehalten wird. An dem Kopf 6 abgewandten Endbereich ist auf den Schaft 7 ein Handgriff 9 zur besseren Handhabung der Mittel zum Einschrauben 5 aufgesteckt.

Wie aus Fig. 2 ersichtlich ist, werden mit diesen Mitteln 5 zum Einschrauben die Pedikelschrauben 1 in den jeweils entsprechend vorbereiteten Wirbelkörper 2 eingeschraubt werden.

Aus Fig. 3 ist ersichtlich, dass der Handgriff 9 vom Schaft 7, mit welchem er in bekannter Weise über Klinkmittel verbunden war, gelöst und abgenommen worden ist. Der Schaft 7 bleibt weiterhin mit dem U-förmigen Kopfteil 4 der Pedikelschraube 1 verbunden. Auf den Schaft 7 lässt sich eine Führungshülse 10 aufstecken, welche mit ersten Führungsmitteln 11 ausgestattet sind.

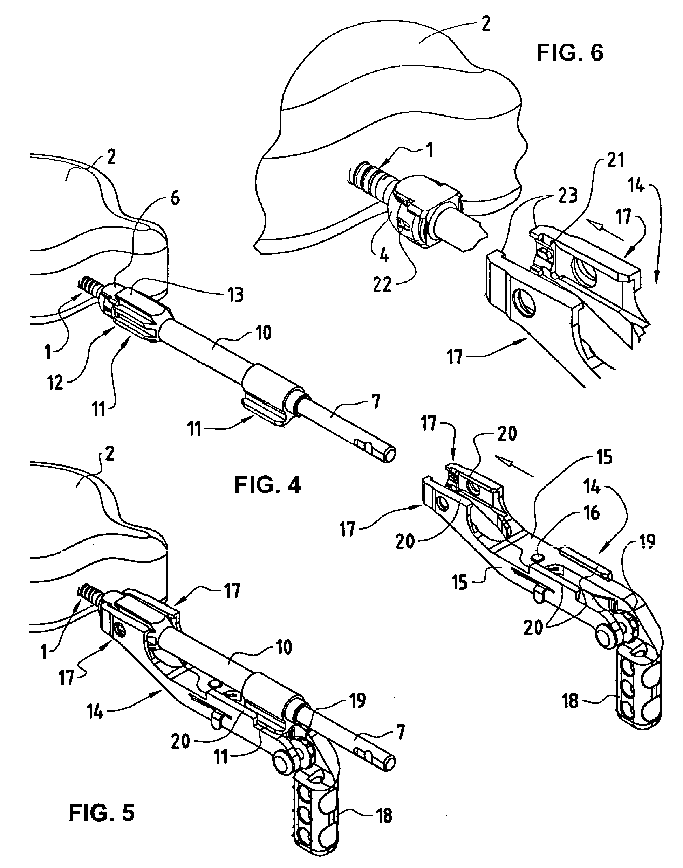

Fig. 4 zeigt die auf den Schaft 7 aufgesteckte Führungshülse 10. Die Führungshülse 10 ist am vorderen Endbereich 12 mit zwei vorstehenden Laschen 13 ausgestattet, zwischen welche beiden Laschen 13 genau der Kopf 6 hinein passt. Dadurch wird die Winkellage der Führungshülse 10 bezüglich des Kopfes 6 und somit der Pedikelschraube 1 definiert. Um anzuzeigen, dass die Führungshülse 10 bezüglich des Schaftes 7 bzw. dem Kopf 6 und somit der Pedikelschraube 1 die korrekte Position eingenommen hat, können an der Führungshülse 10 und am Schaft 7 in bekannter Weise nicht dargestellte Markierungen angebracht werden, wodurch eine visuelle Kontrolle der exakten Position der Führungshülse 10 bezüglich der Pedikelschraube 1 ermöglicht wird.

Auf diese ersten Führungsmittel 11 der Führungshülse 10 ist eine Zange 14 aufsetzbar, wie nachfolgend noch im Detail beschrieben wird. Diese Zange 14 umfasst zwei Hebel 15, die über eine Schwenkachse 16 gegeneinander verschwenkbar sind. An den vorderen Enden der Hebel 15 ist jeweils eine Backe 17 angebracht, am hinteren Ende des einen Hebels 15 ist ein Handgriff 18 angeformt, zwischen den beiden hinteren Enden der Hebel 15 sind bekannte Arretiermittel 19 angebracht. Die Zange 14 ist mit zweiten Führungsmitteln 20 ausgestattet, die Zange 14 lässt sich somit über die zweiten Führungsmittel 20 geführt über die ersten Führungsmittel 11 der Führungshülse 10 auf diese Führungshülse 10 aufschieben, der aufgeschobene Zustand ist in Fig. 5 dargestellt.

Wie aus der vergrösserten Darstellung gemäss Fig. 6 ersichtlich ist, sind die Backen 17 der Zange 14 so ausgebildet, dass diese die beiden Schenkel des U-förmigen Kopfteils 4 der Pedikelschraube 1 aussenseitig greifen können. Die Backen 17 der Zange 14 sind mit Nocken 21 versehen, die entsprechend an den beiden Schenkeln des U-förmigen Kopfteils 4 angebrachte Ausnehmungen 22 eingreifen können. Zusätzlich sind die Backen 17 der Zange 14 mit Vorsprüngen 23 ausgestattet, die die Schenkel des U-förmigen Kopfteils 4 derart umschliessen, dass zusammen mit den Nocken 21, die in die Ausnehmungen 22 eindringen, ein Verdrehen und Verkippen der Zange bezüglich der Pedikelschraube 1 verunmöglicht wird.

Im voll aufgeschobenen Zustand der Zange 14 auf die Führungshülse 10 bzw. auf den U-förmigen Kopfteil 4 der Pedikelschraube 1, wie dies in Fig. 5 dargestellt ist, können die Arretiermittel 19 betätigt werden, die Zange 14 ist dadurch mit der Pedikelschraube 1 fest verbunden.

Wie aus den Fig. 4 bis 6 ersichtlich ist, sind die Backen 17 der Zange 14 bezüglich der Schwenkachse 16 der Zange 14 in Schwenkachsenrichtung versetzt angeordnet. Das heisst, dass die Zange 14 im auf die Pedikelschraube 1 aufgesetzten und gekoppelten Zustand in axialer Richtung gegen die Pedikelschraube 1 einen Freiraum gewährt, so dass der Zugang auf die Pedikelschraube 1 zwischen den Backen 17 hindurch in axialer Richtung vollumfänglich gewährleistet ist. Die korrekte Position der Zange 14 bezüglich der Führungshülse 10 und entsprechend der Pedikelschraube 1 kann wiederum in bekannter Weise durch an der Führungshülse 10 und der Zange 14 angebrachte, nicht dargestellte Markierungen visuell angezeigt werden.

Nachdem die Zange 14 in der vorgängig beschriebenen Art an die Pedikelschraube 1 angekoppelt ist, dargestellt in Fig. 5, können die Führungshülse 10 und der Schaft 7 von der Pedikelschraube 1 in axialer Richtung abgezogen werden, geführt durch die Führungsmittel 11, die mit den zweiten Führungsmitteln 20 zusammenwirken.

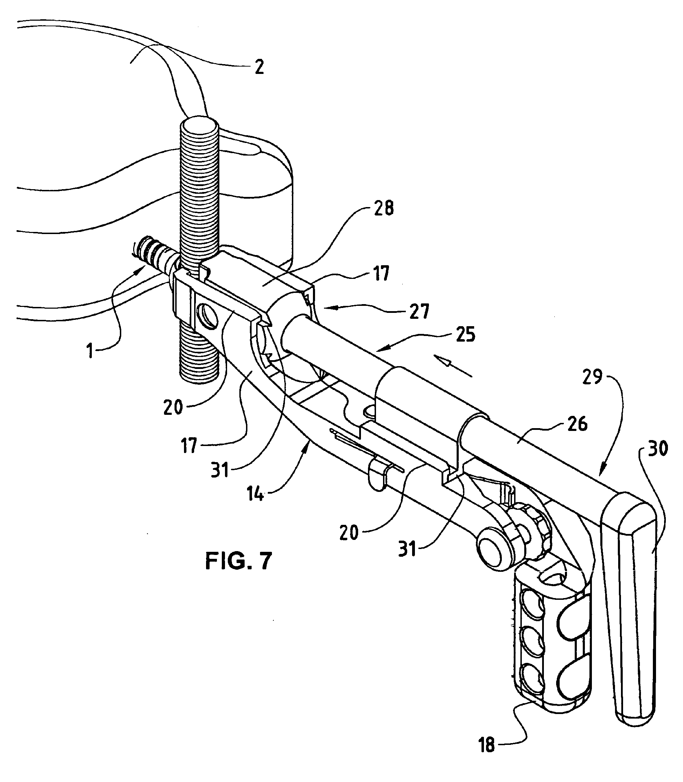

Wie aus Fig. 7 ersichtlich ist, kann in den Freiraum zwischen den beiden Backen 17 der Zange 14 der in die Pedikelschraube 1 einzusetzende Stab 24 gelegt werden, über Mittel 25 zum Einsetzen des Stabes 24 in die Pedikelschraube 1 kann danach der Stab 24 in den U-förmigen Kopfteil 4 der Pedikeischraube 1 eingedrückt werden. Diese Mittel 25 zum Einsetzen des Stabes sind aus einer Stange 26 gebildet, an deren vorderen Ende 27 ein Einpressteil 28 und an deren hinteren Ende 29 ein Handgriff 30 angebracht ist. Die Stange 26 ist mit dritten Führungsmitteln 31 versehen, diese dritten Führungsmittel 31 werden beim Einsetzen des Stabes 24 in den U-förmigen Kopfteil 4 der Pedikelschraube 1 in die zweiten Führungsmittel 20 der Zange 14 eingeführt, somit sind die Mittel 25 zum Einsetzen des Stabes in die Pedikelschraube beim Einsetzvorgang bezüglich der Pedikelschraube 1 ebenfalls exakt geführt. Zum Einsetzen des Stabes 24 in den U-förmigen Kopfteil 4 der Pedikelschraube können der Handgriff 18 der Zange 14 und der Handgriff 30 der Mittel 25 zum Einsetzen des Stabes 24 gegeneinander gedrückt werden, sodass möglichst keine Kräfte auf den Wirbelkörper 2, in welchen die entsprechende Pedikelschraube 1 eingeschraubt ist, ausgeübt werden.

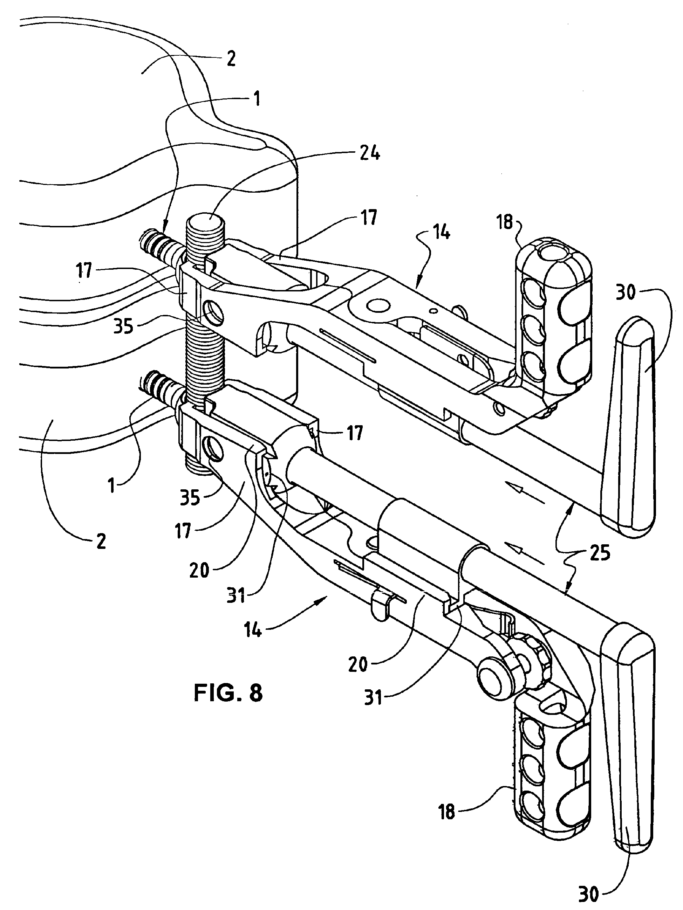

Aus Fig. 8 sind zwei Wirbelkörper 2 ersichtlich, die zu stabilisieren sind. In jeden dieser Wirbelkörper 2 ist eine Pedikelschraube 1 eingeschraubt, gemäss der vorgängig beschriebenen Vorgehensweise. Auf beide Pedikelschrauben 1 ist jeweils eine Zange 14 aufgesetzt und mit der Pedikelschraube 1 gekoppelt. In den Freiraum zwischen den Backen 17 der beiden Zangen 14 ist der in die Pedikelschrauben 1 einzusetzende Stab 24 eingelegt worden, über die Mittel 25 zum Einsetzen des Stabes 24 in die Pedikelschrauben 1 ist danach der Stab 24 in die Pedikelschrauben 1 eingedrückt worden, indem die beiden Mittel 25 zum Einsetzen des Stabes 24, geführt durch die zweiten Führungsmittel 20 der Zange 14 und der dritten Führungsmittel 31 der Mittel 25 zum Einsetzen des Stabes 24 gegen die Pedikelschrauben 1 gedrückt worden sind.

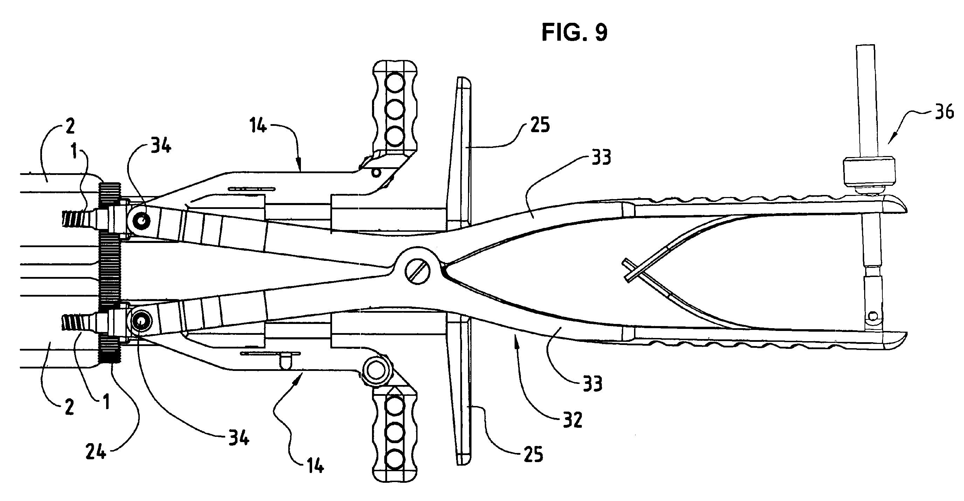

Bevor der Stab 24 in die Pedikelschrauben 1 eingesetzt wird, wie dies zu Fig. 8 beschrieben worden ist, können die beiden Wirbelkörper 2 distrahiert werden, d.h. die gewünschte Einstellung der Wirbelstellung wird vorgenommen. Hierzu kann, wie dies in Fig. 9 dargestellt ist, ein Distrahierwerkzeug 32 eingesetzt werden, das aus zwei gelenkig miteinander verbundenen Hebeln 33 gebildet ist. Am vorderen Ende dieser Hebel 33 sind kopfförmige Erhebungen 34 angebracht, die in entsprechende Bohrungen 35 (Fig. 8) eingesetzt werden können, die seitlich an den Backen 17 der Zangen 14 vorgesehen sind. Am hinteren Ende der Hebel 33 ist eine bekannte Einstellvorrichtung 36 angebracht, mit welchem die vorderen Enden der Hebel 33 zusammengedrückt oder aufgespreizt werden können. Dadurch kann die gewünschte Wirbelstellung eingestellt werden. Durch die Ausgestaltung der kopfförmigen Erhebungen 34 und der Bohrungen 35 kann das Distrahierwerkzeug 32 in die Zangen 14 eingeklinkt werden, ein Verdrehen der Zangen 14 und der entsprechenden Pedikelschrauben 1 wird dadurch vermieden. Wenn die Wirbelkörper 2 in die gewünschte Stellung gebracht worden sind, kann die erforderliche Länge des in die Pedikelschrauben 1 einzusetzenden Stabes 24 durch Messen festgelegt werden, der einzusetzende Stab kann auf die entsprechende Länge gebracht werden, und danach in die Pedikelschrauben 1 eingesetzt werden, wie dies vorgängig zu Fig. 8 beschrieben worden ist.

Im hier dargestellten Ausführungsbeispiel wird ein elastischer Stab eingesetzt, der aus einem Material auf Polyurethanbasis besteht, und dessen Oberfläche Rippen und Rillen aufweist. Der Aufnahmeteil des U-förmigen Kopfteils 4 ist ebenfalls mit entsprechenden Rippen und Rillen ausgestattet, der in die Pedikelschrauben 1 eingesetzte Stab 24 ist im eingesetzten Zustand somit formschlüssig gehalten, ein Verschieben der Pedikelschrauben 1 in Längsrichtung des Stabes wird dadurch ausgeschlossen. Selbstverständlich können aber auch andere Materialien und Oberflächenbeschaffenheiten für den Stab und den Aufnahmebereich des U-förmigen Kopfteils der Pedikelschraube eingesetzt werden.

Nachdem der Stab 24 in die Pedikelschrauben 1 eingesetzt ist, können die Mittel 25 zum Einsetzen des Stabes aus der Zange 14 entlang der zweiten Führungsmittel 20 herausgezogen werden.

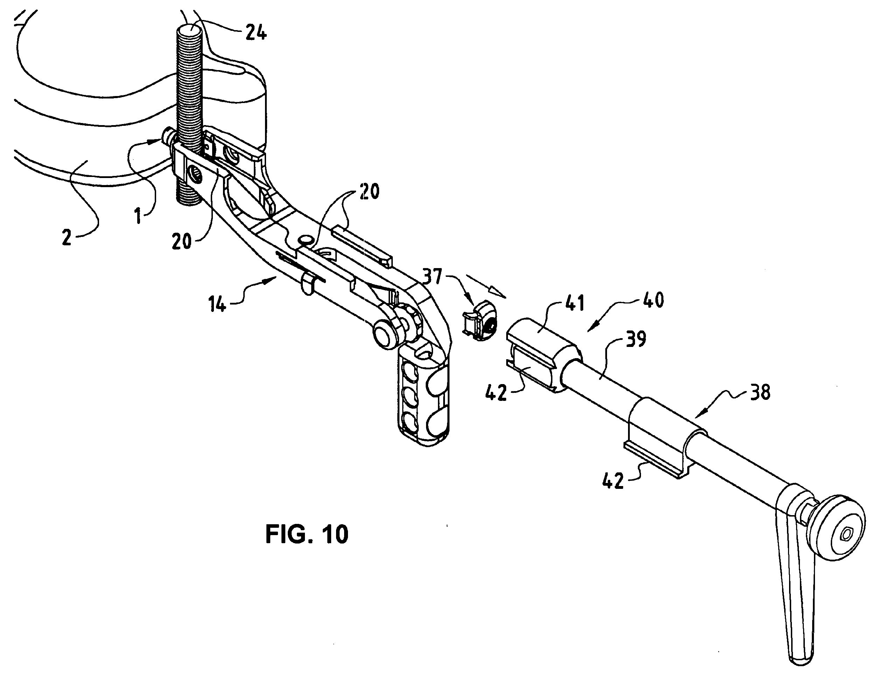

Auf die Pedikelschrauben 1, in welche der Stab 24 eingesetzt worden ist, kann nun die Verschlusseinrichtung 37 aufgesetzt werden, wozu Mittel 38 zum Aufsetzen und Verspannen eingesetzt werden, wie dies aus Fig. 10 ersichtlich ist. Die Mittel 38 zum Aufsetzen und Verspannen der Verschlusseinrichtung 37 auf die Pedikelschraube 1 sind aus einer Hülse 39 gebildet, an deren vorderen Ende 40 ein Aufnahmeteil 41 für die Aufnahme der Verschlusseinrichtung 37 angebracht ist. Diese Hülse 39 ist mit vierten Führungsmitteln 42 ausgestattet, die mit den zweiten Führungsmitteln 20 der Zange 14 zusammenwirken können, wie nachfolgend noch beschrieben wird.

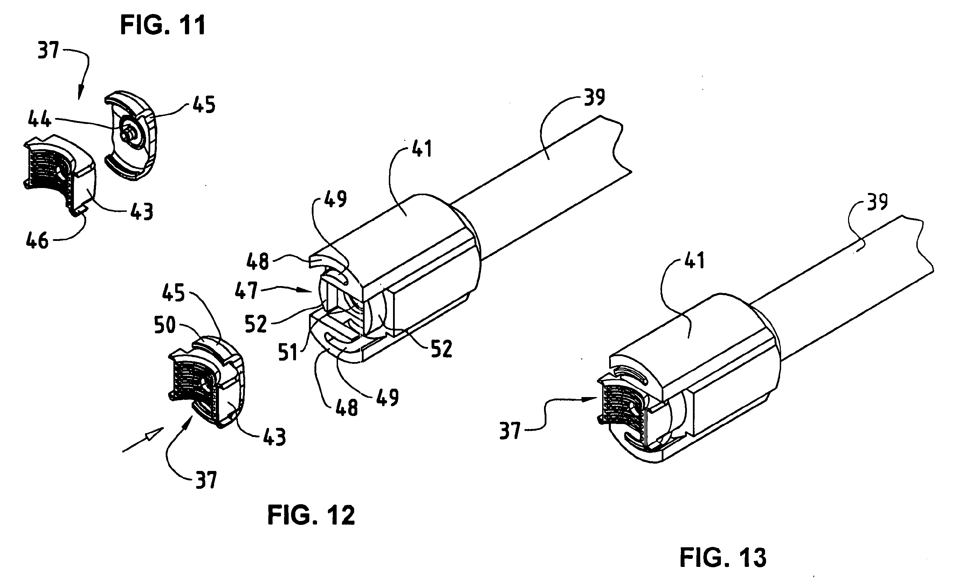

Fig. 11 zeigt den Aufbau einer bekannten Verschlusseinrichtung 37, wie sie in der europäischen Patentanmeldung mit der Anmeldenummer 07150489.8 im Detail beschrieben ist. Diese Verschlusseinrichtung umfasst einen Einführteil 43, eine Spannschraube 44 und einen Verriegelungsteil 45. Der Einführteil 43 lässt sich zwischen die Schenkel des U-förmigen Kopfteils 4 aufsetzen, der Verriegelungsteil 45 wird dann um die Spannschraube 44 bezüglich des Einführteils 43 gedreht, wobei die Nocken 46 in bekannter Weise in entsprechende Schlitze an der Pedikelschraube 1 eingreifen, danach wird die Spannschraube 44 angezogen, der Einführteil 43 wird gegen den in die Pedikelschraube 1 eingesetzten Stab gepresst, der Stab wird dadurch in optimaler Weise in der Pedikeischraube gehalten.

Der Bereich des Aufnahmeteils 41, welcher der Aufnahme der Verschlusseinrichtung 37 dient, ist aus Fig. 12 ersichtlich. Die vormontierte Verschlusseinrichtung 37 wird in die verriegelte Position gebracht, das heisst, dass der Verriegelungsteil 45 bezüglich des Einführteils 43 aus der in Fig. 12 dargestellten Position um 90° gedreht wird. Die Verschlusseinrichtung 37 befindet sich dadurch im Verriegelungszustand. Die Verschlusseinrichtung 37 kann in die schlitzförmige Aufnahme 47 des Aufnahmeteils 41 eingeführt werden. Die beiden Backen 48, die die schlitzförmige Aufnahme 47 bilden, sind jeweils mit einer schlitzförmigen Ausnehmung 49 versehen. In der in die schlitzförmige Aufnahme 47 eingesetzten Position kann der Verriegelungsteil 45 wieder zurückgedreht werden, die Flanken 50 des Verriegelungsteils 45 gelangen dann in die schlitzförmigen Ausnehmungen 49 des Aufnahmeteils 41, die Verschlusseinrichtung 37 wird dadurch im Aufnahmeteil 41 der Mittel 38 zum Aufsetzen und Verspannen der Verschlusseinrichtung festgehalten, wie dies aus Fig. 13 ersichtlich ist.

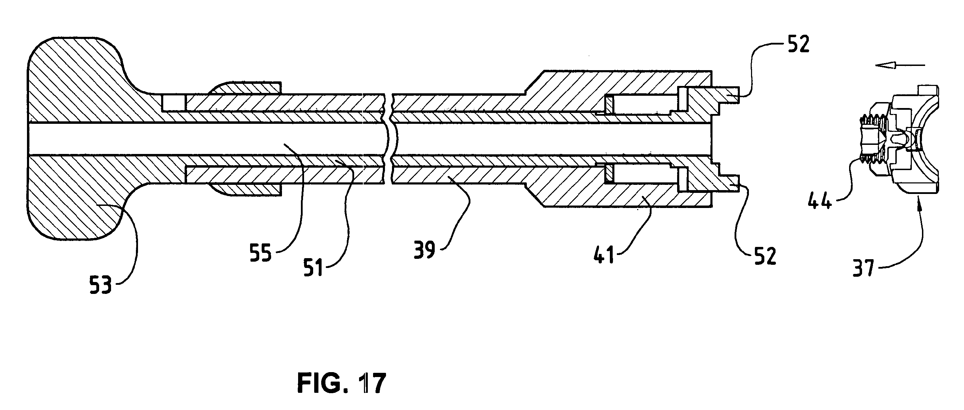

Zum Verdrehen des Verriegelungsteils 45 der Verschlusseinrichtung 37 im in den Aufnahmeteil 41 der Mittel 38 zum Aufsetzen und Verspannen eingesetzten Zustand ist in die Hülse 39 ein Drehelement 51 eingesetzt, wie dies insbesondere aus Fig. 17 ersichtlich ist. Das vordere Ende dieses Drehelementes 51 ist mit Vorsprüngen 52 ausgestattet, welche im eingesetzten Zustand der Verschlusseinrichtung 37 in den Aufnahmeteil 41 über den Verriegelungsteil 45 vorstehend sind. Durch Verdrehen dieses Drehelementes 51 kann der Verriegelungsteil 45 bezüglich des Einführteils 43 der Verschlusseinrichtung 37 verdreht werden. Diese am Drehelement 51 angebrachten Vorsprünge 52 sind auch in Fig. 12 sichtbar.

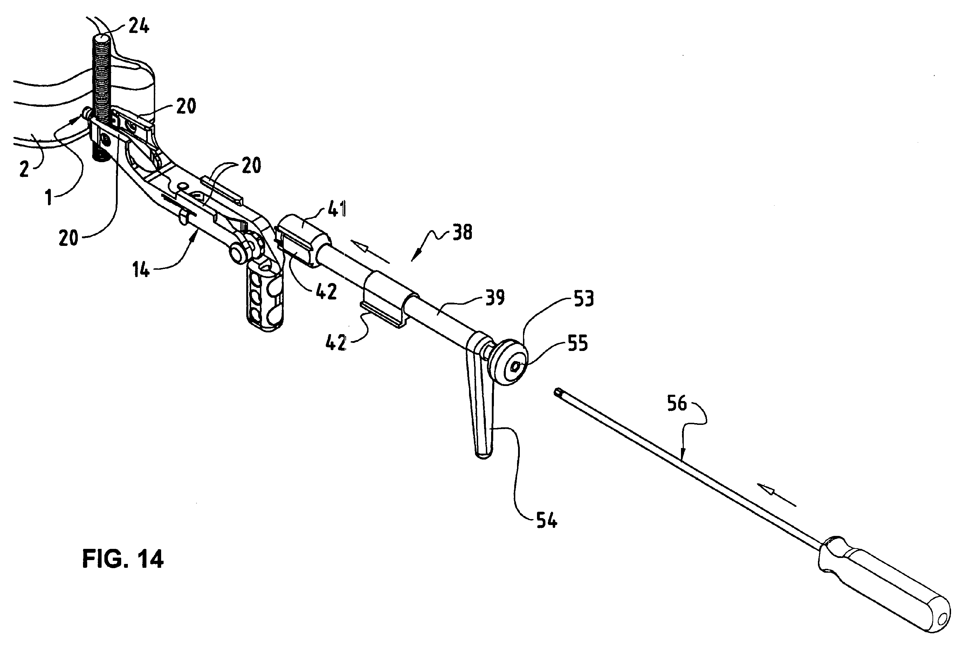

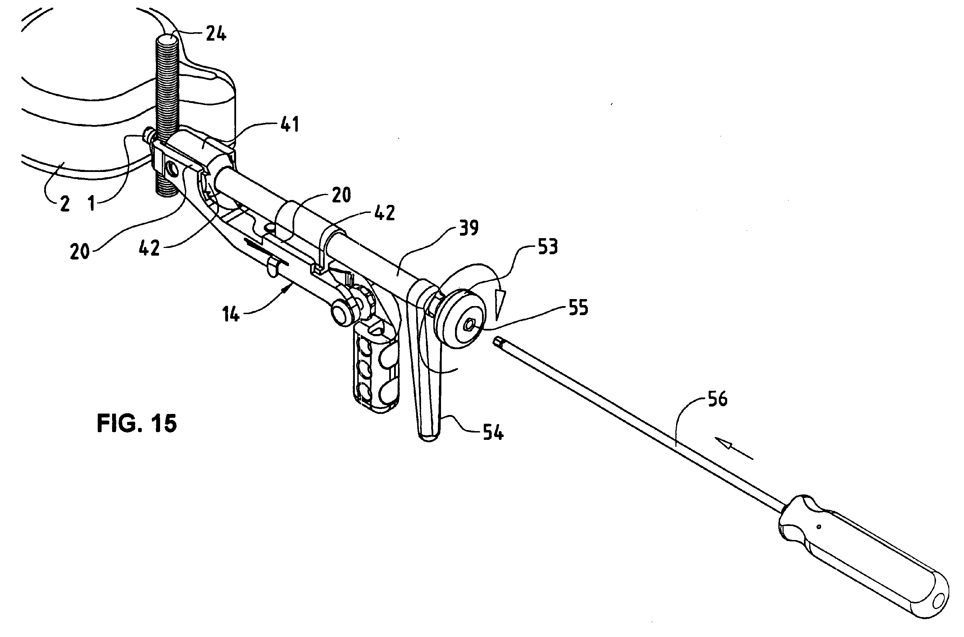

Die Mittel 38 zum Aufsetzen und Verspannen der Verschlusseinrichtung 37 auf die Pedikelschraube 1 werden nun in axialer Richtung gegen die Pedikelschraube 1 geführt, wobei die vierten Führungsmittel 42 in die zweiten Führungsmittel 20 einfahren, wie dies aus den Fig. 14 und 15 ersichtlich ist. Zur besseren Handhabung ist am hinteren Ende der Hülse 39 ein Handgriff 53 angebracht. Durch die exakte Führung der Hülse 39 bezüglich der Zange 14 wird die im Aufnahmeteil 41 gehaltene Verschlusseinrichtung 37 exakt auf den U-förmigen Kopfteil 4 der Pedikelschraube 1 aufgesetzt. Der Einführteil 43 der Verschlusseinrichtung 37 wird zwischen die beiden Schenkel des U-förmigen Kopfteils 4 der Pedikelschraube 1 eingeführt, der Verriegelungsteil 45 kann durch Verdrehen des Drehelementes 51 bezüglich der Pedikelschraube 1 in die verriegelte Position gebracht werden. Hierzu ist das Drehelement 51 am hinteren Ende mit einem Drehkopf 53 ausgestattet. Durch nicht dargestellte, an der Hülse 39 und an der Zange 14 angebrachte Markierungen kann wiederum visuell angezeigt werden, wenn die exakte Position der Verschlusseinrichtung 37 bezüglich der Pedikelschraube 1 eingenommen ist. Entsprechend kann auch die verriegelte Position des Verriegelungsteils 45 durch nicht dargestellte Markierungen visuell dargestellt werden. Damit auch während dieses Vorgangs möglichst keine Kräfte auf den Wirbelkörper 2, in welchen die entsprechende Pedikelschraube 1 eingeschraubt ist, ausgeübt werden, können auch hier beim Einsetzen der Verschlusseinrichtung 37 und beim Verriegeln der Handgriff 18 der Zange 14 und der Handgriff 53 der Hülse 39 gegeneinander gedrückt werden.

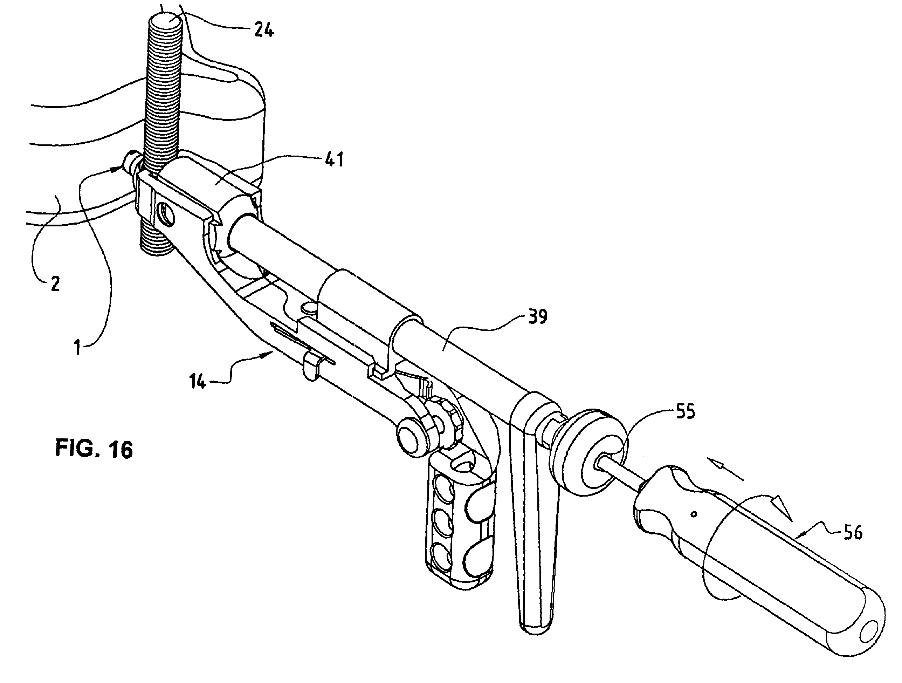

Wie insbesondere aus Fig. 17 entnehmbar ist, ist das Drehelement 51 mit einer durchgehenden Längsbohrung 55 versehen. In diese Längsbohrung 55 lässt sich ein Schraubenzieher 56 einstecken, wie dies aus den Fig. 14 bis 16 ersichtlich ist. Mit diesem Schraubenzieher 56 kann die Spannschraube 44 (Fig. 17) in der Verschlusseinrichtung 37, die auf die Pedikelschraube aufgesetzt und sich in der verriegelten Position befindet, verdreht werden, dadurch wird der Einführteil 43 gegen den in die Pedikelschraube eingesetzten Stab 24 gepresst. Die Verschlusseinrichtung 37 befindet sich damit im verriegelten und gespannten Zustand auf der Pedikelschraube 1, der Stab 24 wird dabei in optimalster Weise in der Pedikelschraube gehalten.

Die Mittel 38 zum Aufsetzen und Verspannen der Verschlusseinrichtung 37 werden danach aus der Zange 14, die nach wie vor mit der Pedikelschraube 1 verkoppelt ist, geführt durch die zweiten Führungsmittel 20 der Zange und die vierten Führungsmittel 42 der Hülse 39, ausgefahren, die Arretiermittel 19 der Zange 14 (Fig. 4 und 5) können gelöst werden, die Zange 14 kann von der Pedikelschraube 1 entfernt werden.

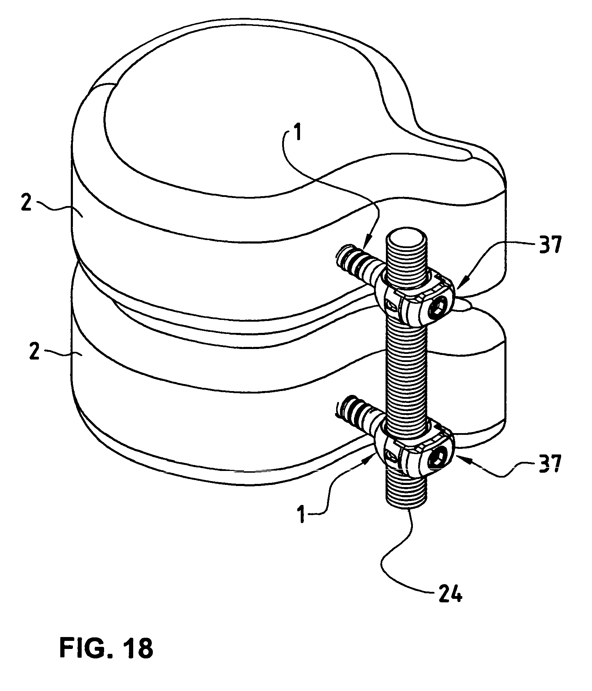

Aus Fig. 18 ist das Stabilisierungssystem für eine Wirbelsäule im fertig eingesetzten Zustand ersichtlich. Die Pedikelschrauben 1 sind in die Wirbelkörper 2 eingeschraubt, der in die Pedikelschrauben 1 eingesetzte Stab 24 wird durch die Verschlusseinrichtungen 37 in optimaler Weise in den Pedikelschrauben 1 gehalten, die beiden Wirbelkörper 2 sind dadurch in optimaler Weise stabilisiert. Selbstverständlich werden für die Stabilisierung von Wirbelkörpern einer Wirbelsäule zwei Stabilisierungssysteme eingesetzt, die symmetrisch angeordnet sind.

Die ersten Führungsmittel 11, die zweiten Führungsmittel 20, die dritten Führungsmittel 31 und die vierten Führungsmittel 42 können in bekannter Weise aus zueinander passenden Rippen und Rillen gebildet sein, selbstverständlich sind auch andere Führungsmittel denkbar, beispielsweise Führungsstangen und Hülsen.

Dieser erfindungsgemässe Instrumentensatz ermöglicht ein exaktes Einsetzen eines Stabilisierungssystems in die Wirbelsäule eines Körpers, alle einzusetzenden Instrumente werden exakt geführt, die einzusetzenden Elemente des Stabilisierungssystems können dadurch auf sehr exakte Weise in die richtige Position gebracht werden, ein ungenaues Platzieren dieser Elemente ist praktisch ausgeschlossen, was einem Chirurgen, der eine entsprechende Operation durchführt, sehr entgegen kommt.

## Patentansprüche

1. Instrumentensatz zum Einsetzen eines Stabilisierungssystems, betstehend aus Verankerungselementen (1), die jeweils eine Schraubenhalterung (3) und einen U-förmigen Kopfteil (4) aufweisen, in welchen Kopfteil (4) ein Stab (24) zur Stabilisierung einsetzbar ist und mit einer Verschlusseinrichtung (37) verschliessbar und verriegelbar ist, in die Wirbelsäule eines Körpers, umfassend Mittel (5) zum Einsetzen der Verankerungselemente (1) in den jeweiligen Knochen- oder Wirbelkörper (2), Mittel (25) einschliesslich einer Zange (14) zum Einsetzen des Stabes (24) zur Stabilisierung in die eingesetzten Verankerungselemente (1), Mittel (38) zum Aufsetzen und Verspannen der Verschlusseinrichtungen (37) auf die Verankerungselemente (1), **dadurch gekennzeichnet, dass** die Mittel (5) zum Einsetzen der Verankerungselemente (1) einen Kopf (6) und einen Schaft (7) aufweisen, dass der Kopf (6) derart ausgebildet ist, dass er in den U-förmigen Kopfteil (4) der Verankerungselemente (1) einsteckbar ist, dass der Schaft (7) mit ersten Führungsmitteln (11) ausgestattet ist, entlang welchen die mit zweiten Führungsmitteln (20) ausgerüstete Zange (14) geführt auf den Kopfteil (4) des Verankerungselements (1) aufsetzbar und mit diesem koppelbar ist, und dass die Mittel (25) zum Einsetzen des Stabes (24) und die Mittel (38) zum Aufsetzen und Verspannen der Verschlusseinrichtung (37) mit weiteren Führungsmitteln (31; 42) ausgestattet sind, die mit den zweiten Führungsmitteln (20) der Zange (14) zusammenwirken.

2. Instrumentensatz nach Anspruch 1, **dadurch gekennzeichnet, dass** die ersten Führungsmittel (11) an einer Führungshülse (10) angebracht sind, welche Führungshülse (10) auf den Schaft (7) aufschiebbar ist.

3. Instrumentensatz nach Anspruch 1 oder 2, **dadurch gekennzeichnet, dass** die Zange (14) mit Backen (17) versehen ist, welche die beiden Schenkel des U-förmigen Kopfteils (4) aussenseitig greifen, und welche mit Nocken (21) versehen sind, die in entsprechend an den beiden Schenkeln angebrachte Ausnehmungen (22) eingreifen und **dadurch** die Zange (14) an das Verankerungselement (1) angekoppelt ist.

4. Instrumentensatz nach Anspruch 3, **dadurch gekennzeichnet, dass** die Backen (17) der Zange (14) bezüglich der Schwenkachse (16) der Zange (14) in Schwenkachsenrichtung versetzt angeordnet sind.

5. Instrumentensatz nach einem der Ansprüche 1 bis 4, **dadurch gekennzeichnet, dass** die Zange (14) in der mit dem Verankerungselement (1) gekoppelten Position über Arretiermittel (19) arretierbar ist.

6. Instrumentensatz nach einem der Ansprüche 1 bis 5, **dadurch gekennzeichnet, dass** die Mittel (25) zum Einsetzen des Stabes (24) in das Verankerungselement (1) aus einer Stange (26) gebildet sind, an deren vorderen Ende (27) ein Einpressteil (28) und an deren hinteren Ende (29) ein Handgriff (30) angebracht ist, und dass die Stange (26) mit dritten Führungsmitteln (31) versehen ist, welche mit den zweiten Führungsmitteln (20) der Zange (14) zusammenwirken.

7. Instrumentensatz nach einem der Ansprüche 1 bis 6, **dadurch gekennzeichnet, dass** die Mittel (38) zum Aufsetzen und Verspannen der Verschlusseinrichtung (37) aus einer Hülse (39) gebildet sind, an deren vorderen Ende (40) ein Aufnahmeteil (41) für die Aufnahme der Verschlusseinrichtung (37) angebracht ist, und welche Hülse (39) mit vierten Führungsmitteln (42) ausgestattet ist, die mit den zweiten Führungsmitteln (20) der Zange (14) zusammenwirken.

8. Instrumentensatz nach einem der Ansprüche 1 bis 7, **dadurch gekennzeichnet, dass** die Verschlusseinrichtung aus einem Einführteil (43), einem Verriegelungsteil (45) und einer Spannschraube (44) besteht.

9. Instrumentensatz nach Anspruch 8, **dadurch gekennzeichnet, dass** der Aufnahmeteil (41) mit schlitzförmigen Ausnehmungen (49) versehen ist, in welche der Verriegelungsteil (45) der Verschlusseinrichtung (37) eindrehbar ist und **dadurch** die Verschlusseinrichtung (37) im Aufnahmeteil (41) gehalten ist.

10. Instrumentensatz nach Anspruch 8 oder 9, **dadurch gekennzeichnet, dass** in die Hülse (39) ein Drehelement (51) eingesetzt ist, dessen vorderes Ende mit Vorsprüngen (52) ausgestattet ist, welche im eingesetzten Zustand der Verschlusseinrichtung (37) in den Aufnahmeteil (41) über den Verriegelungsteil (45) vorstehend sind und **dadurch** der Verriegelungsteil (45) bezüglich des Einführteils (43) durch Verdrehen des Drehelementes (51) verdrehbar ist.

11. Instrumentensatz nach einem der Ansprüche 7 bis 10, **dadurch gekennzeichnet, dass** die Hülse (39) an deren hinteren Ende mit einem Handgriff (54) versehen ist.

12. Instrumentensatz nach Anspruch 10 oder 11, **dadurch gekennzeichnet, dass** am Drehelement (51) am über die Hülse (39) vorstehenden hinteren Ende ein Drehknopf (53) angebracht ist.

13. Instrumentensatz nach einem der Ansprüche 10 bis 12, **dadurch gekennzeichnet, dass** das Drehelement (51) eine durchgehende Längsbohrung (55) aufweist, und dass in die durchgehende Längsbohrung (55) ein Schraubenzieher (56) einsteckbar ist, mit welchem die Spannschraube (44) der Verschlusseinrichtung (37) verdrehbar ist.

## Claims

1. Instrument set for inserting a stabilization system, comprising anchoring elements (1), each having one screw-type holder (3) and one U-shaped head part (4), into which head part (4) a rod (24) is insertable for stabilization and is lockable and latchable by means of a locking device (37), into the spinal column of a body, comprising means (5) for inserting the anchoring elements (1) into the respective bone or vertebral bodies (2), means (25), including a pair of pliers (14), for inserting the stabilization rod (24) into the inserted anchoring elements (1), and means (38) for placement and tensioning of the locking devices (37) on the anchoring elements (1), **characterised in that** the means (5) for inserting the anchoring elements (1) have a head (6) and a shaft (7), that the head (6) is so constructed that it is insertable into the U-shaped head part (4) of the anchoring elements (1), that the shaft (7) is equipped with first guide means (11), along which the pliers (14) equipped with second guide means (20) are able to be guided onto the head part (4) of the anchoring element (1) and are able to be coupled thereto, and that the means (25) for insertion of the rod (24) and the means (38) for placement and tensioning the locking device (37) are equipped with further guide means (31; 42) that co-operate with the second guide means (20) of the pliers (14).

2. Instrument set according to claim 1, **characterised in that** the first guide means (11) are attached to a guide sleeve (10), which guide sleeve (10) is able to be slid on the shaft (7).

3. Instrument set according to claim 1 or 2, **characterised in that** the pliers (14) are provided with jaws (17), which grasp the two branches of the U-shaped head part (4) on the outside, and which have protrusions (21), which engage in recesses (22) correspondingly provided on the two branches and thereby couple the pliers (14) to the anchoring element (1).

4. Instrument set according to claim 3, **characterised in that** the jaws (17) of the pliers (14) are disposed in a way offset relative to the pivot axis (16) of the pliers (14) in the direction of the pivot axis.

5. Instrument set according to one of the claims 1 to 4, **characterised in that** via arresting means (19) the pliers (14) are able to be locked in place in the position in which they are coupled to the anchoring element (1).

6. Instrument set according to one of the claims 1 to 5, **characterised in that** the means (25) for inserting the rod (24) into the anchoring element (1) are formed from a bar (26), on whose front end (27) a press-in part (28) and on whose rear end (29) a handle (30) is attached, and that the bar (26) is provided with third guide means (31), which co-operate with the second guide means (20) of the pliers (14).

7. Instrument set according to one of the claims 1 to 6, **characterised in that** the means (38) for placement and tensioning of the locking device (37) are formed from a sleeve (39), on whose front end (40) a seating part (41) is fixed to receive the locking device (37), and which sleeve (39) is equipped with fourth guide means (42), which co-operate with the second guide means (20) of the pliers (14).

8. Instrument set according to one of the claims 1 to 7, **characterised in that** the locking device consists of an insertion part (43), a latching part (45) and a clamping bolt (44).

9. Instrument set according to claim 8, **characterised in that** the seating part (41) is provided with slot-like recesses (49), into which the latching part (45) of the locking device (37) can be rotated, and the locking device (37) is thereby held in the seating part (41).

10. Instrument set according to claim 8 or 9, **characterised in that** a rotating element (51) is inserted into the sleeve (39), the front end of which rotating element (51) being equipped with projections (52) which, in the inserted state of the locking device (37), protrude into the seating part (41) over the latching part (45), and the latching part (45) is thereby rotatable relative to the insertion part (43) by turning of the rotating element (51).

11. Instrument set according to one of the claims 7 to 10, **characterised in that** the sleeve (39) is provided with a handle (54) on its rear end.

12. Instrument set according to claim 10 or 11, **characterised in that** attached to the rotating element (51) on the rear end projecting over the sleeve (39) is a turning knob (53).

13. Instrument set according to one of the claims 10 to 12, **characterised in that** the rotating element (51) has a continuous longitudinal bore hole (55), and that insertable into the continuous longitudinal bore hole (55) is a screwdriver (56), with which the clamping bolt (44) of the locking device (37) is able to be rotated.

## Revendications

1. Ensemble d'instruments destiné à installer un système de stabilisation, comprenant des éléments d'ancrage (1), chacun ayant une fixation à vis (3) et une partie de tête en forme de U (4), partie de tête (4) dans laquelle une tige (24) est susceptible d'être insérée pour la stabilisation et est susceptible d'être verrouillée et enclenchée par un dispositif de verrouillage (37), dans la colonne vertébrale d'un corps, comprenant des moyens (5) pour insérer lesdits éléments d'ancrage (1) dans les corps osseux ou vertébraux respectifs (2), des moyens (25), y compris une pince (14), pour insérer ladite tige de stabilisation (24) dans lesdits éléments d'ancrage insérés (1), et des moyens (38) pour le placement et le serrage desdits dispositifs de serrage (37) sur lesdits éléments d'ancrage (1), **caractérisé en ce que** lesdits moyens (5) pour insérer lesdits éléments d'ancrage (1) ont une tête (6) et un fût (7), que ladite tête (6) est formée de manière à être susceptible d'être insérée dans la partie de tête en forme de U (4) desdits éléments d'ancrage (1), que ledit fût (7) est équipé de premiers moyens de guidage (11), le long desquels ladite pince (14), équipée de seconds moyens de guidage (20), est susceptible d'être placée de manière guidée sur la partie de tête (4) de l'élément d'ancrage (1) et est susceptible d'être couplée à ce dernier, et que lesdits moyens (25) pour insérer ladite tige (24) et lesdits moyens (38) pour le placement et le serrage du dispositif de verrouillage (37) sont équipés de moyens de guidage supplémentaires (31; 42) qui coopèrent avec les seconds moyens de guidage (20) de ladite pince (14).

2. Ensemble d'instruments selon la revendication 1, **caractérisé en ce que** lesdits premiers moyens de guidage (11) sont attachés à une douille de guidage (10) susceptible d'être glissée sur ledit fût (7).

3. Ensemble d'instruments selon la revendication 1 ou 2, **caractérisé en ce que** ladite pince (14) est munie de becs (17), qui saisissent les deux bras de ladite partie de tête en forme de U (4) du côté extérieur, et qui ont des saillies (21) qui viennent s'engager dans des évidements (22) prévus de manière correspondante sur les deux bras, et, de ce fait, viennent coupler la pince (14) à l'élément d'ancrage (1).

4. Ensemble d'instruments selon la revendication 3, **caractérisé en ce que** les becs (17) de ladite pince (14) sont agencés de manière décalée par rapport à l'axe de pivotement (16) de la pince (14) dans la direction de l'axe de pivotement.

5. Ensemble d'instruments selon l'une des revendications 1 à 4, **caractérisé en ce que** ladite pince (14) est susceptible d'être verrouillée, par des moyens d'arrêt (19), dans la position où elle est couplée audit élément d'ancrage (1).

6. Ensemble d'instruments selon l'une des revendications 1 à 5, **caractérisé en ce que** lesdits moyens (25) pour insérer ladite tige (24) dans l'élément d'ancrage (1) sont formés d'une barre (26) dont l'extrémité avant (27) est dotée d'une partie à enfoncement (28) et dont l'extrémité arrière (29) est dotée d'un manche (30), et que ladite barre (26) est munie de troisièmes moyens de guidage (31) qui coopèrent avec les seconds moyens de guidage (20) de ladite pince (14).

7. Ensemble d'instruments selon l'une des revendications 1 à 6, **caractérisé en ce que** lesdits moyens (38) pour le placement et le serrage du dispositif de verrouillage (37) sont formés d'une douille (39) dont l'extrémité avant (40) est dotée d'une partie de siège (41) pour recevoir ledit dispositif de verrouillage (37), douille (39) qui est équipée (39) de quatrièmes moyens de guidage (42) qui coopèrent avec les seconds moyens de guidage (20) de ladite pince (14).

8. Ensemble d'instruments selon l'une des revendications 1 à 7, **caractérisé en ce que** ledit dispositif de verrouillage est constitué d'une pièce d'insertion (43), d'une pièce à enclenchement (45) et d'un boulon de serrage (44).

9. Ensemble d'instruments selon la revendication 8, **caractérisé en ce que** ladite partie de siège (41) est munie d'évidements en forme de fente (49) dans lesquels la pièce à enclenchement (45) dudit dispositif de verrouillage (37) peut être introduite par rotation, et ledit dispositif de verrouillage (37) est ainsi retenu dans ladite partie de siège (41).

10. Ensemble d'instruments selon la revendication 8 ou 9, **caractérisé en ce qu'**un élément de rotation (51) est inséré dans ladite douille (39), l'extrémité avant dudit élément de rotation (51) étant équipée de saillies (52) qui, dans l'état inséré dudit dispositif de verrouillage (37), font saillie dans la partie de siège (41) sur ladite pièce à enclenchement (45), et la pièce à enclenchement (45) est ainsi susceptible d'être pivotée par rapport à ladite pièce d'insertion (43) en faisant pivoter ledit élément de rotation (51).

11. Ensemble d'instruments selon l'une des revendications 7 à 10, **caractérisé en ce que** ladite douille (39) est munie d'un manche (54) à son extrémité arrière.

12. Ensemble d'instruments selon la revendication 10 ou 11, **caractérisé en ce qu'**un bouton tournant (53) est attaché audit élément de rotation (51) à son extrémité arrière faisant saillie au-delà de ladite douille (39).

13. Ensemble d'instruments selon l'une des revendications 10 à 12, **caractérisé en ce que** ledit élément de rotation (51) a un alésage longitudinal continu (55), et que, dans ledit alésage longitudinal continu (55), un tournevis (56) peut être inséré avec lequel le boulon de serrage (44) dudit dispositif de verrouillage (37) peut être tourné.
